# EUROPÄISCHE PATENTSCHRIFT

(11) **EP 0 504 819 B1**
(45) Veröffentlichungstag und Bekanntmachung des Hinweises auf die Patenterteilung: **02.07.1997**
(21) Anmeldenummer: 92104614.0
(22) Anmeldetag: 17.03.1992
(51) Int. Cl.: C12P 17/12, C12N 1/20

(54) **Mikrobiologisches Verfahren zur Herstellung von 6-Hydroxynikotinsäure**
Microbiological process for the preparation of 6-hydroxy nicotinic acid
Procédé microbiologique pour la préparation de l'acide 6-hydroxy nicotinique

(30) Priorität: 18.03.1991 CH 811/91
(43) Veröffentlichungstag der Anmeldung: 23.09.1992
(73) Patentinhaber: LONZA AG, CH-3945 Gampel/Wallis (CH)
(72) Erfinder: Kiener, Andreas, Dr., Visp (Kanton Wallis) (CH)
(74) Vertreter: Weinhold, Peter, Dr.

(56) Entgegenhaltungen:
- EP-A- 0 152 948
- EP-A- 0 187 680
- APPLIED AND ENVIRONMENTAL MICROBIOLOGY Bd. 54, Nr. 7, 1988, WASHINGTON Seiten 1766 - 1769 TORU NAGASAWA ET AL. 'Nitrile hydratase-catalyzed production of nicotinamide from 3-cyanopyridine in Rhodococcus rhodochrous J1.'

## Beschreibung

Die Erfindung betrifft ein neues mikrobiologisches Verfahren zur Herstellung von 6-Hydroxynikotinsäure ausgehend von 3-Cyanpyridin sowie neue für das Verfahren geeignete Mikroorganismen.

6-Hydroxynikotinsäure ist ein wichtiges Zwischenprodukt zur Herstellung von 5,6-Dichlornikotinsäure (CH-PS 664 754), welche wiederum ein Ausgangsprodukt für pharmazeutische Wirkstoffe darstellt (Setcliff et al., J.of Chem. and Eng. Data, Vol.21, Nr.2, (1976), S.246).

In EP-A-0 152 948 wird ein mikrobiologisches Verfahren zur Herstellung von 6-Hydroxynikotinsäure beschrieben: 6-Hydroxynikotinsäure wird durch enzymatische Hydroxylierung von Nikotinsäure in Gegenwart eines Mikroorganismus der Gattung Pseudomonas, Bacillus oder Achromobacter hergestellt.

Bisher sind, ausgehend von 3-Cyanpyridin, weder chemische noch mikrobiologische Verfahren zur Herstellung von 6-Hydroxynikotinsäure bekannt.

Aufgabe der vorliegenden Erfindung war es, ein einfaches mikrobiologisches Verfahren zur Herstellung von 6-Hydroxynikotinsäure, ausgehend von 3-Cyanpyridin, zur Verfügung zu stellen.

Diese Aufgabe wurde mit einem neuen Verfahren gemäss Patentanspruch 3, das mit neuen Mikroorganismen gemäss Patentanspruch 1 durchgeführt wird, gelöst.

Erfindungsgemäss sind alle Mikroorganismen geeignet, die befähigt sind mit 3-Cyanpyridin als einziger Kohlenstoff-, Stickstoff- und Energiequelle zu wachsen und dieses als Substrat in 6-Hydroxynikotinsäure zu überführen.

Diese Mikroorganismen sind Bestandteil der Erfindung und können mit Hilfe üblicher mikrobiologischer Techniken beispielsweise aus Kläranlagen mit 3-Cyanpyridin als Wachstumssubstrat selektioniert und isoliert werden.

Der Begriff "Mikroorganismen, die befähigt sind, mit 3-Cyanpyridin als einziger Kohlenstoff-, Stickstoff- und Energiequelle zu wachsen", umfasst sowohl Mischungen von Mikroorganismen als auch Rein-Isolate dieser Mikroorganismen, die unter sterilen oder unsterilen Fermantationsbedingungen eingesetzt werden.

Zweckmässig werden die Mikroorganismen Achromobacter sp., die im folgenden aufgrund detaillierterer Identifizierungsdaten als Agrobacterium sp. bezeichnet werden, und deren Deszendenten und Mutanten eingesetzt. Diese wurden bei der Deutschen Sammlung für Mikroorganismen und Zellkulturen GmbH (DSM), Mascheroderweg 1b, D-3300 Braunschweig am 31.1.1991 mit der Bezeichnung DSM 6336 hinterlegt.

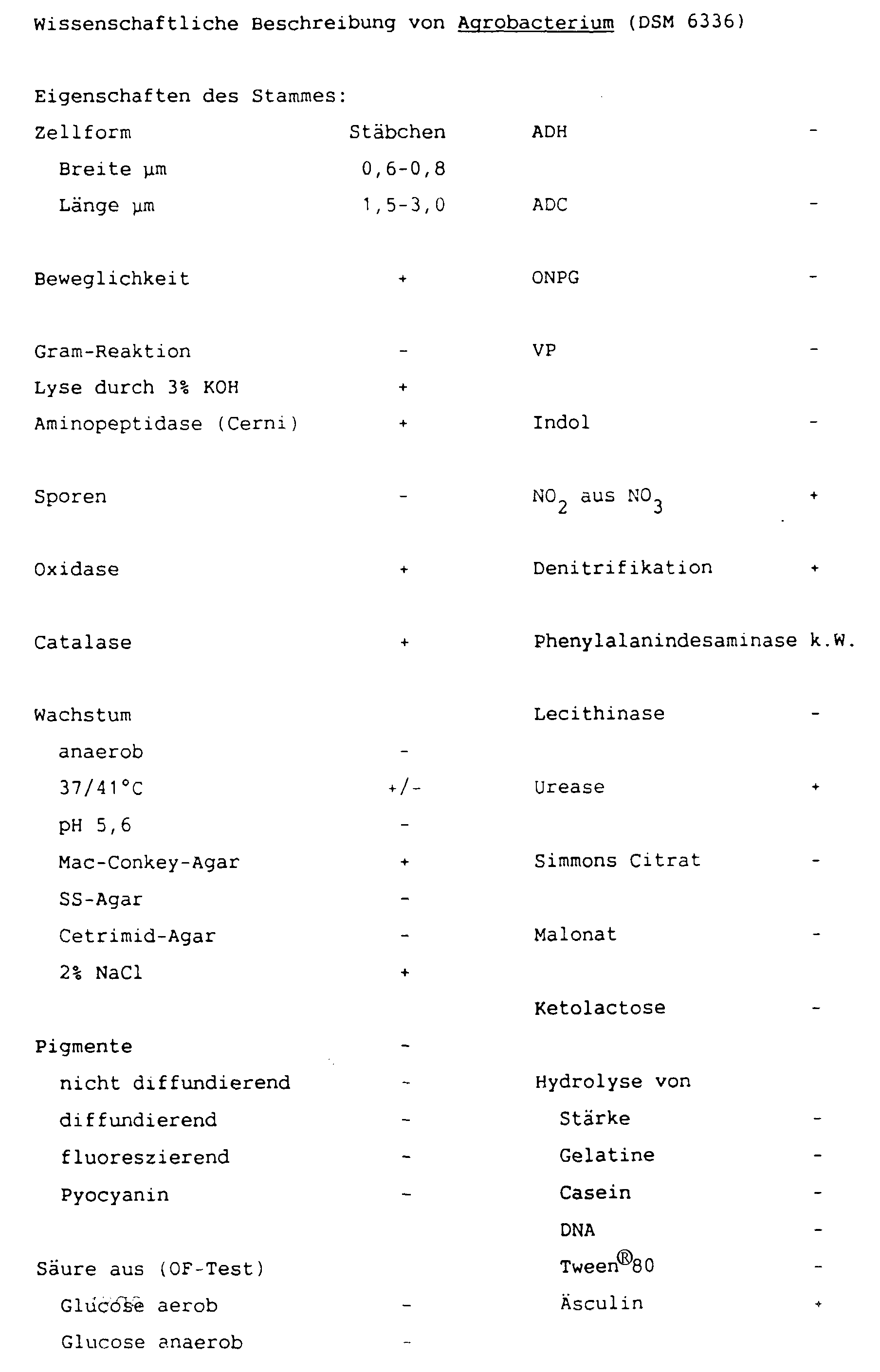

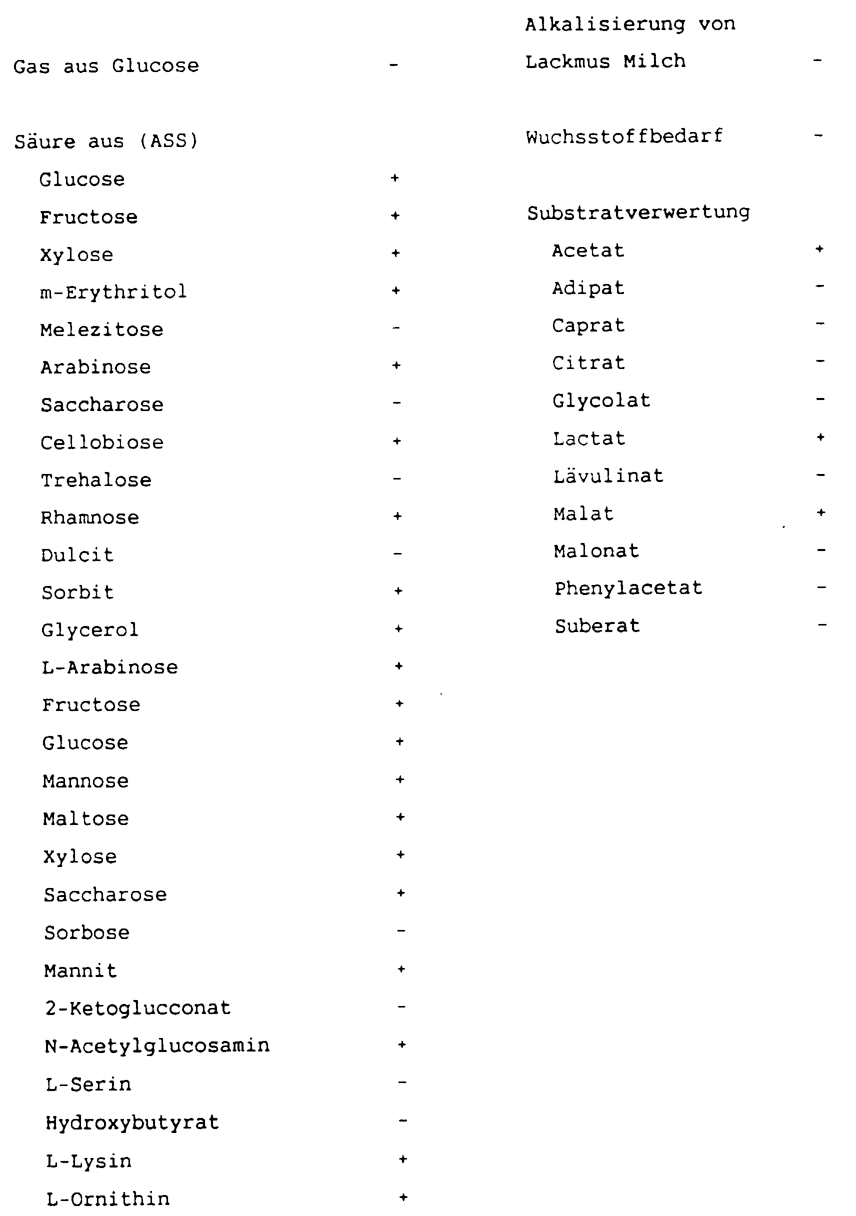

Das Verfahren zur Herstellung von 6-Hydroxynikotinsäure wird erfindungsgemäss derart durchgeführt, dass man 3-Cyanpyridin mit einem dieser Mikroorganismen zu 6-Hydroxynikotinsäure biotransformiert, wobei letzteres im Medium akkumuliert wird.

Vor der eigentlichen Umsetzung werden diese Mikroorganismen üblicherweise kultiviert (angezogen) und die wirksamen Enzyme der Mikroorganismen zweckmässig mit 3-Cyanpyridin induziert.

Üblicherweise erfolgt die Kultivierung (Anzucht) und die Induktion mit 3-Cyanpyridin in einer Konzentration von 0,01 bis 20 Gew.%, vorzugsweise in einer Konzentration von 0,1 bis 1 Gew.%.

Anschliessend können die Mikroorganismen entweder vor der Substratzugabe (3-Cyanpyridin) mittels üblichen Trennverfahren geerntet werden, oder das Substrat (3-Cyanpyridin) kann direkt zu den Mikroorganismen zugegeben werden.

Für das eigentliche Verfahren wird dann zweckmässig die Zellsuspension auf eine optische Dichte bei 650 nm von 1 bis 100, vorzugsweise auf eine optische Dichte von 5 bis 80, eingestellt.

Als Medium können die in der Fachwelt üblichen angewendet werden, vorzugsweise wird eines der Medien verwendet, deren Zusammensetzung in Tabelle 1 und 2 angegeben ist.

Das Substrat (3-Cyanpyridin) kann zur Produktion von 6-Hydroxynikotinsäure einmalig oder kontinuierlich zugegeben werden.

Zweckmässig erfolgt die Substratzugabe so, dass die Substratkonzentration im Medium 20 Gew.%, vorzugsweise so, dass die Substratkonzentration 10 Gew.% nicht übersteigt.
Üblicherweise erfolgt die Umsetzung von 3-Cyanpyridin zur 6-Hydroxynikotinsäure mit ruhenden Zellen.

Der pH-Wert der Umsetzung liegt zweckmässig in einem Bereich von 4 bis 10, vorzugsweise in einem Bereich von 5 bis 9.

Zweckmässig wird die Umsetzung bei einer Temperatur von 10 bis 50°C, vorzugsweise bei einer Temperatur von 20 bis 40°C,durchgeführt.

Nach einer üblichen Umsetzungsdauer von 1 bis 100 Stunden kann 6-Hydroxynikotinsäure beispielsweise durch Ansäuern der zellfreien Fermentationslösung isoliert werden.

### Beispiel 1

### Isolierung von 3-Cyanpyridin-metabolisierenden Mikroorganismen

Aerobe 3-Cyanpyridin-metabolisierende Mikroorganismen wurden im A+N-Medium (Tabelle 1) mit Zusatz von 0,1% (w/v) 3-Cyanpyridin als einzige Kohlenstoff- und Energiequelle angereichert. Die allgemeinen Techniken zur Isolation von Mikroorganismen sind beispielsweise in "G.Drews, Mikrobiologisches Praktikum, 4.Aufl., Springer Verlag, 1983" beschrieben. Als Inokolum wurden Proben aus Kläranlagen verwendet.

Die Anreicherungen wurden in Schüttelkolben bei 30°C angezogen. Nach dreimaligem Überimpfen in frisches Medium wurden die Anreicherungen auf dem gleichen Medium mit Zusatz von 16 g Agar pro Liter ausgestrichen und bei 30°C inkubiert. Nach mehrmaligem Ausstreichen auf Agarmedium konnten Reinkulturen isoliert werden.

**Tabelle 1:**

| A+N-Medium | |
|---|---|
| Zusammensetzung: | Konzentration (mg/l) |
| (NH₄)₂SO₄ | 2000 |
| Na₂HPO₄ | 2000 |
| KH₂PO₄ | 1000 |
| NaCl | 3000 |
| MgCl₂·6H₂O | 400 |
| CaCl₂·2H₂O | 14.5 |
| FeCl₃·6H₂O | 0.8 |
| Pyridoxal-Hydrochlorid | 10·10⁻³ |
| Riboflavin | 5·10⁻³ |
| Nicotinsäureamid | 5·10⁻³ |
| Thiamin-Hydrochlorid | 2·10⁻³ |
| Biotin | 2·10⁻³ |
| Panthothensäure | 5·10⁻³ |
| p-Aminobenzoat | 5·10⁻³ |
| Folsäure | 2·10⁻³ |
| Vitamin B12 | 5·10⁻³ |
| ZnSO₄.7H₂o | 100·10⁻³ |
| MnCl₂.4H₂O | 90·10⁻³ |
| H₃BO₃ | 300·10⁻³ |
| CoCl₂.6H₂O | 200·10⁻³ |
| CuCl₂.2H₂O | 10·10⁻³ |
| NiCl₂.6H₂O | 20·10⁻³ |
| Na₂MoO₄.2H₂O | 30·10⁻³ |
| EDTANa₂.2H₂O | 5·10⁻³ |
| FeSO₄.7H₂O | 2·10⁻³ |
| (pH der Lösung wurde auf 7.0 eingestellt) | |

### Beispiel 2

### Umsetzung von 3-Cyanpyridin zu 6-Hydroxynikotinsäure

a) Agrobacterium sp. DSM-Nr. 6336 wurde im A+N-Medium (Tabelle 1) unter Zusatz von 0,1% (w/v) 3-Cyanpyridin in einem Fermenter bei pH 7 und bei einer Temperatur von 30°C angezogen. Anschliessend wurden die Zellen abzentrifugiert , im A+N-Medium resuspendiert und auf eine optische Dichte bei 650 nm von 10 eingestellt.
   Diese Zellsuspension wurde in einen Schüttelkolben gegeben und mit 0,1 mol/l (10,4 g/l) 3-Cyanpyridin versetzt. Nach einer Inkubation von 16 h bei 30°C auf einer Schüttelmaschine konnten mit analytischen Methoden 0,06 mol/l (8,3 g/l) 6-Hydroxynikotinsäure in der zellfreien Lösung nachgewiesen werden, was eine Ausbeute von 66%, bezogen auf eingesetztes 3-Cyanpyridin,entsprach.
b) Agrobacterium sp. DSM-Nr. 6336 wurde in einem Mineralsalzmedium (Tabelle 2) unter Zusatz von 0,1% (w/v) 3-Cyanpyridin in einem Fermenter (Arbeitsvolumen 5,5 l) bei pH 7 und bei einer Temperatur von 30°C angezogen. Zur pH-Regelung wurde eine Lösung bestehend aus 1 mol/l Schwefelsäure und 2 mol/l 3-Cyanpyridin und eine Lösung aus 3 mol/l Natriumhydroxid hinzugegeben.
   Nach 20 h Wachstum (Kultivierung) betrug die optische Dichte bei 650 nm 5,0, wobei weder 3-Cyanpyridin noch 6-Hydroxynikotinsäure nachweisbar war.

Zu diesem Zeitpunkt wurde 3-Cyanpyridin (100 g, 1 mol) in den Fermenter gegeben.
Nach einer weiteren Inkubation von 6 h wurde nochmals 3-Cyanpyridin (100 g, 1 mol) zugegeben.
Nach weiteren 12 h wurde diese Mikroorganismensuspension (Biomasse) abzentrifugiert und der Überstand auf pH 2,0 angesäuert, um die 6-Hydroxynikotinsäure auszufällen.
Insgesamt wurden 269 g 6-Hydroxynikontinsäure isoliert, entsprechend einer Ausbeute von 96%,bez. auf eingesetztes 3-Cyanpyridin.

**Tabelle 2:**

| Zusammensetzung des Minersalzmediums | |
|---|---|
| | |
| - MgCl₂·6H₂O | 0,8 g/l |
| - CaCl₂ | 0,16 g/l |
| - Na₂SO₄ | 0,25 g/l |
| - KH₂PO₄ | 0,4 g/l |
| - Na₂HPO₄ | 0,9 g/l |
| - SLF | 1 ml/l |
| - FeEDTA | 15 ml/l |
| | |

| Zusammensetzung der Spurenelemente (SLF) im Mineralsalzmedium | |
|---|---|
| | |
| - KOH | 15 g/l |
| - EDTANa₂·2H₂O | 100 g/l |
| - ZnSO₄ ·7H₂O | 9 g/l |
| - MnCl₂·4H₂O | 4 g/l |
| - H₃BO₃ | 2,7 g/l |
| - CoCl₂·6H₂O | 1,8 g/l |
| - CuCl₂·2H₂O | 1,5 g/l |
| - NiCl₂.6H₂O | 0,18 g/l |
| - Na₂MoO₄·2H₂O | 0,2 g/l |
| | |

| Zusammensetzung von FeEDTA: | |
|---|---|
| | |
| - EDTA Na₂·2H₂O | 5 g/l |
| - FeSO₄·7H₂O | 2 g/l |
| (Der pH der Lösung wurde auf 7,0 eingestellt) | |

## Patentansprüche (Patentansprüche für folgende(n) Vertragsstaat(en): AT, BE, CH, LI, DE, FR, GB, IT, NL, SE)

1. Mikroorganismen, dadurch gekennzeichnet, daß sie befähigt sind, mit 3-Cyanpyridin als einziger Kohlenstoff-, Stickstoff- und Energiequelle zu wachsen und dieses als Substrat in 6-Hydroxynikotinsäure zu überführen.

2. Mikroorganismen nach Anspruch 1 mit der Bezeichnung Agrobacterium sp., hinterlegt bei der DSM mit der Nummer 6336, und deren Deszendenten und Mutanten, die befähigt sind, mit 3-Cyanpyridin als einziger Kohlenstoff-, Stickstoff- und Energiequelle zu wachsen und dieses als Substrat in 6-Hydroxynikotinsäure zu überführen.

3. Mikrobiologisches Verfahren zur Herstellung von 6-Hydroxynikotinsäure, dadurch gekennzeichnet, daß man 3-Cyanpyridin mit Mikroorganismen gemäß einem der Ansprüche 1 und 2 zu 6-Hydroxynikotinsäure biotransformiert, wobei letztere im Medium akkumuliert wird.

4. Verfahren nach Anspruch 3, dadurch gekennzeichnet, daß die wirksamen Enzyme der Mikroorganismen mit 3-Cyanpyridin induziert werden.

5. Verfahren nach mindestens einem der Ansprüche 3 und 4, dadurch gekennzeichnet, daß die Umsetzung unter einmaliger oder kontinuierlicher Substratzugabe erfolgt, so daß die Substratkonzentration 20 Gew.-% nicht übersteigt.

6. Verfahren nach mindestens einem der Ansprüche 3 bis 5, dadurch gekennzeichnet, daß man die Umsetzung bei einem pH von 4 bis 10 und einer Temperatur von 10 bis 50°C durchführt.

## Patentansprüche (Patentansprüche für folgende(n) Vertragsstaat(en): ES)

1. Mikrobiologisches Verfahren zur Herstellung von 6-Hydroxynikotinsäure, dadurch gekennzeichnet, daß man 3-Cyanpyridin mit Mikroorganismen, die befähigt sind, mit 3-Cyanpyridin als einziger Kohlenstoff-, Stickstoff- und Energiequelle zu wachsen und dieses als Substrat in 6-Hydroxynikotinsäure zu überführen, zu 6-Hydroxynikotinsäure biotransformiert, wobei letztere im Medium akkumuliert wird.

2. Verfahren nach Anspruch 1, dadurch gekennzeichnet, daß es sich bei den Mikroorganismen um Mikroorganismen mit der Bezeichnung Agrobacterium sp., hinterlegt bei der DSM mit der Nummer 6336, oder deren Deszendenten und Mutanten handelt.

3. Verfahren nach mindestens einem der Ansprüche 1 und 2, dadurch gekennzeichnet, daß die wirksamen Enzyme der Mikroorganismen mit 3-Cyanpyridin induziert werden.

4. Verfahren nach mindestens einem der Ansprüche 1 bis 3, dadurch gekennzeichnet, daß die Umsetzung unter einmaliger oder kontinuierlicher Substratzugabe erfolgt, so daß die Substratkonzentration 20 Gew.-% nicht übersteigt.

5. Verfahren nach mindestens einem der Ansprüche 1 bis 4, dadurch gekennzeichnet, daß man die Umsetzung bei einem pH von 4 bis 10 und einer Temperatur von 10 bis 50°C durchführt.

## Claims (Claims for the following Contracting State(s): AT, BE, CH, LI, DE, FR, GB, IT, NL, SE)

1. Microorganisms, characterized in that they are capable of growing with 3-cyanopyridine as the sole carbon, nitrogen and energy source and of converting said compound, as a substrate, to 6-hydroxynicotinic acid.

2. The microorganisms according to claim 1 with the designation Agrobacterium sp., deposited at the DSM with the deposit number 6336, and the descendants and mutants thereof, said microorganisms being capable of growing with 3-cyanopyridine as the sole carbon, nitrogen and energy source and of converting said compound, as a substrate, to 6-hydroxynicotinic acid.

3. A microbiological process for the preparation of 6-hydroxynicotinic acid, characterized in that 3-cyanopyridine is biotransformed with microorganisms according to one of the claims 1 and 2 to 6-hydroxynicotinic acid, wherein the latter one is accumulated in the medium.

4. The process according to claim 3, characterized in that the effective enzymes of the microorganisms are induced with 3-cyanopyridine.

5. The process according to at least one of the claims 3 and 4, characterized in that the reaction takes place under substrate addition once or continuously so that the substrate concentration does not exceed 20 % by weight.

6. The process according to at least one of the claims 3 to 5, characterized in that the reaction is performed at a pH of 4 to 10 and a temperature of 10 to 50 °C.

## Claims (Claims for the following Contracting State(s): ES)

1. A microbiological process for the preparation of 6-hydroxynicotinic acid, characterized in that 3-cyanopyridine, by means of microorganisms which are capable of growing with 3-cyanopyridine as the sole carbon, nitrogen and energy source and of converting 3-cyanopyridine as a substrate to 6-hydroxynicotinic acid, is biotransformed to 6-hydroxynicotinic acid, wherein the latter one is accumulated in the medium.

2. The process according to claim 1, characterized in that the microorganisms are microorganisms with the designation Agrobacterium sp., deposited at the DSM with the deposit number 6336, or the descendants and mutants thereof.

3. The process according to at least one of the claims 1 and 2, characterized in that the effective enzymes of the microorganisms are induced with 3-cyanopyridine.

4. The process according to at least one of the claims 1 to 3, characterized in that the reaction takes place under substrate addition once or continuously so that the substrate concentration does not exceed 20 % by weight.

5. The process according to at least one of the claims 1 to 4, characterized in that the reaction is performed at a pH of 4 to 10 and a temperature of 10 to 50 °C.

## Revendications (Revendications pour l'(les) Etat(s) contractant(s) suivant(s): AT, BE, CH, LI, DE, FR, GB, IT, NL, SE)

1. Microorganismes caractérisés en ce qu'ils sont aptes à pousser avec la 3-cyanopyridine en tant que seule et unique source de carbone, d'azote et d'énergie et de transformer celle-ci en qualité de substrat en acide 6-hydroxynicotinique.

2. Microorganismes selon la revendication 1, portant la désignation Agrobacterium sp., déposés auprès du DSM sous le n° 6336 et avec ses descendants et mutants qui sont aptes à pousser avec la 3-cyanopyridine en tant que seule et unique source de carbone, d'azote et d'énergie et de transformer celle-ci en qualité de substrat en l'acide 6-hydroxynicotinique.

3. Procédé microbiologique pour la préparation de l'acide 6-hydroxynicotinique, caractérisé en ce que l'on transforme biologiquement la 3-cyanopyridine avec des microorganismes selon l'une des revendications 1 et 2 en l'acide 6-hydroxynicotinique de sorte que ce dernier s'accumule dans le milieu.

4. Procédé selon la revendication 1, caractérisé en ce que les enzymes efficaces des microorganismes sont induits par la 3-cyanopyridine.

5. Procédé selon au moins l'une des revendications 3 et 4, caractérisé en ce que la réaction s'effectue sous addition de substrat en une seule fois ou de façon continue de façon que la concentration de substrat ne dépasse pas 20 % en poids.

6. Procédé selon au moins l'une des revendications 3 à 5, caractérisé en ce que l'on conduit la réaction à un pH de 4 à 10 et à une température de 10 à 50°C.

## Revendications (Revendications pour l'(les) Etat(s) contractant(s) suivant(s): ES)

1. Procédé microbiologique pour la préparation de l'acide 6-hydroxynicotinique, caractérisé en ce que l'on transforme biologiquement la 3-cyanopyridine avec des microorganismes qui sont aptes à pousser avec la 3-cyanopyridine en tant que seule et unique source de carbone, d'azote et d'énergie et que l'on transforme celle-ci en tant que substrat en l'acide 6-hydroxynicotinique, pour donner l'acide 6-hydroxynicotinique, ce dernier s'accumulant dans le milieu.

2. Procédé selon la revendication 1, caractérisé en ce que les microorganismes sont des microorganismes portant la désignation Agrobacterium sp., déposés auprès du DSM sous le n° 6336 ou leurs descendants et mutants.

3. Procédé selon au moins l'une des revendications 1 et 2, caractérisé en ce que les enzymes efficaces des microorganismes sont induits par la 3-cyanopyridine.

4. Procédé selon au moins l'une des revendications 1 à 3, caractérisé en ce que la réaction s'effectue sous addition de substrat en une seule fois ou de façon continue de façon que la concentration de substrat ne dépasse pas 20 % en poids.

5. Procédé selon au moins l'une des revendications 1 à 4, caractérisé en ce que l'on conduit la réaction à un pH de 4 à 10 et à une température de 10 à 50°C.
